(19) Europäisches Patentamt
European Patent Office
Office européen des brevets

(11) Numéro de publication: **0 060 182**
**B1**

(12) **FASCICULE DE BREVET EUROPEEN**

(45) Date de publication du fascicule du brevet: **16.01.85**

(51) Int. Cl.⁴: **C 07 D 405/12**, A 61 K 31/44

(21) Numéro de dépôt: **82400346.1**

(22) Date de dépôt: **01.03.82**

(54) **Thioalkylamide de l'acide nicotinique 1-oxyde, ses sels, procédé pour leur préparation et compositions pharmaceutiques.**

(30) Priorité: **11.03.81 FR 8104892**

(43) Date de publication de la demande:
**15.09.82 Bulletin 82/37**

(45) Mention de la délivrance du brevet:
**16.01.85 Bulletin 85/3**

(84) Etats contractants désignés:
**AT BE CH DE FR GB IT LI LU NL SE**

(56) Documents cités:
**FR-A-2 360 587**
**FR-A-2 471 376**

(73) Titulaire: **SANOFI, société anonyme, 40, Avenue George V, F-75008 Paris (FR)**

(72) Inventeur: **Nisato, Dino, Viale Golgi 80, Pavia (IT)**
Inventeur: **Boveri, Sergio, Corso Repubblica 48, Tortona (Alessandria) (IT)**

(74) Mandataire: **Gillard, Marie-Louise et al, Cabinet Beau de Loménie 55, Rue d'Amsterdam, F-75008 Paris (FR)**

EP 0 060 182 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention concerne un nouveau thioalkylamide ayant une activité bloquant les récepteurs $H_2$ de l'histamine, ses sels, un procédé pour leur préparation et des compositions pharmaceutiques les renfermant en tant qu'ingrédients actifs.

Après la subdivision des récepteurs de l'histamine en récepteurs $H_1$ (Ash et Schild, «Brit. J. Pharmac. Chemother.», 1966, *27*, 427) et récepteurs $H_2$ (Black *et al.*, «Nature», 1972, *236*, 1972) et la découverte que le bloc sélectif des récepteurs $H_2$ provoque une inhibition de la sécrétion gastrique, de nombreux produits ont été proposés comme antagonistes des récepteurs $H_2$ de l'histamine, ci-après indiqués $H_2$-bloquants. Ainsi les composés ayant reçu les dénominations communes internationales burimamide, métiamide, cimétidine, ranitidine, tiotidine, étintidine, oxmétidine ont fait l'objet d'un grand nombre de publications scientifiques, et l'un d'entre eux, la cimétidine, constitue déjà un outil entre les mains du médecin pour le traitement de la maladie ulcéreuse.

Tous les produits ci-dessus sont caractérisés par la présence dans leur molécule de la structure suivante:

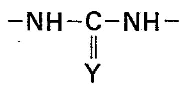
(I)

où Y représente un atome d'oxygène ou de soufre, ou bien un groupe $N-CN$ ou $CH-NO_2$, ladite structure étant linéaire ou incluse dans un cycle comme dans le cas de l'oxmétidine. Les produits ci-dessus sont donc tous caractérisés par la présence de deux atomes d'azote géminaux par rapport à un atome de carbone.

Il est également connu que des récepteurs $H_2$ de l'histamine sont situés non seulement dans la muqueuse gastrique, mais aussi dans le nœud sinusal, dans le myocarde ventriculaire et dans les vaisseaux coronariens et que les $H_2$-bloquants connus sont actifs à la fois sur les récepteurs cardiaques et gastriques. Ainsi, le blocage des récepteurs $H_2$-cardiaques peut être la cause de la bradycardie et de l'asystolie observées comme effets secondaires dans le traitement de la maladie ulcéreuse par la cimétidine («Clinica Terapeutica», 1981, *96*, pp. 81-91, en particulier p. 84).

Il est donc souhaitable de pouvoir disposer de composés présentant une dissociation entre l'activité $H_2$-bloquante gastrique et cardiaque, en faveur de la première, qui soient susceptibles de donner moins d'effets secondaires au niveau cardiaque.

On a maintenant trouvé qu'une nicotinamide 1-oxyde ne présentant pas la structure I ci-dessus possède une bonne action antagonisant les récepteurs $H_2$ de l'histamine et que cette action se produit de préférence au niveau des récepteurs $H_2$-gastriques.

Ainsi la présente invention a pour objet, selon un de ses aspects, un nouveau thioalkylamide, caractérisé par la formule suivante:

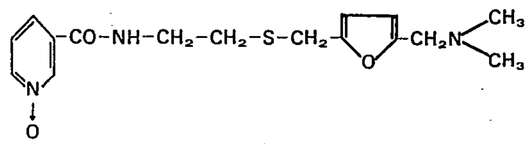
(II)

ainsi que ses sels pharmaceutiquement acceptables.

Les sels pharmaceutiquement acceptables comprennent les sels non toxiques dérivés d'acides minéraux ou organiques salifiant une ou les deux fonctions basiques présentes dans la molécule des composés de formule II, tels que le chlorhydrate, le bromhydrate, le sulfate, le succinate, le tartrate, le citrate, le fumarate, le maléate, le 4,4'-méthylène-bis-(3-hydroxy-2-naphtoate), ci-après désigné pamoate, le 2-naphtalènesulfonate, ci-après désigné napsylate, le méthanesulfonate, ci-après désigné mésylate et le p-toluènesulfonate, ci-après désigné tosylate.

Selon un autre de ses aspects, la présente invention a pour objet un procédé pour la préparation du nouveau composé de formule II ci-dessus, ledit procédé étant caractérisé en ce que l'on traite la 2-(5-diméthylaminométhylfuran-2-ylméthylthio)éthylamine de formule:

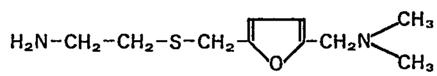
(III)

avec un dérivé fonctionnel de l'acide nicotinique 1-oxyde dans un solvant organique à une température entre 0°C et la température d'ébullition du solvant employé.

Comme dérivé fonctionnel approprié, on peut utiliser l'anhydride, un anhydride mixte, le chlorure ou un ester activé.

La température de réaction peut varier entre 0°C et le point d'ébullition du solvant employé, mais en général on opère à la température ambiante. Il peut être préférable de conduire la réaction au froid lorsqu'elle est exothermique, comme dans le cas où on utilise le chlorure en tant que dérivé fonctionnel de l'acide nicotinique 1-oxyde.

Comme solvant de réaction, on utilise de préférence un alcool, tel que le méthanol ou l'éthanol, ou un solvant halogéné, tel que le chlorure de méthylène, le dichloroéthane ou le chloroforme, mais d'autres solvants organiques compatibles avec les réactifs employés, par exemple le dioxanne, le tétrahydrofuranne ou un hydrocarbure tel que l'hexane peuvent également être employés.

La réaction peut être conduite en présence d'un accepteur de protons, par exemple d'un carbonate alcalin ou d'une amine tertiaire, dans le cas où de l'acide chlorhydrique, ou un autre acide, se libère pendant la réaction, mais cet accepteur de protons n'est pas indispensable pour l'obtention du produit final.

La réaction est assez rapide; en général, après 2 à 4 h à la température ambiante, la réaction est achevée et le thioalkylamide de formule II obtenu est isolé selon les techniques traditionnelles sous forme de base libre ou d'un de ses sels.

La base libre peut être transformée en un de ses sels pharmacetiquement acceptables par traitement avec une solution de l'acide convenable dans un solvant organique, et le sel ainsi obtenu est isolé selon les techniques habituelles, en général par simple filtration du précipité.

Si, à la fin de la réaction, le thioalkylamide de formule II est isolé sous forme de sel, on peut préparer la base libre correspondante en la libérant à l'aide d'un hydroxyde ou d'un carbonate alcalin.

Le nouveau composé de formule II de la présente invention ainsi que ses sels pharmaceutiquement acceptables agissent comme antagonistes sélectifs des récepteurs $H_2$ de l'histamine en inhibant sélectivement la sécrétion gastrique au niveau des récepteurs $H_2$-gastriques avec une faible activité sur les récepteurs $H_2$-cardiaques, et sont donc utiles pour le traitement de la maladie ulcéreuse.

La sélectivité de l'activité des produits de la présente invention vers les récepteurs du type $H_2$ est confirmée par l'absence d'activité du type $H_1$, anticholinergique et papavérinique, dans le test de la contraction provoquée par l'histamine sur l'iléon isolé de cobaye.

L'activité antagoniste des composés de la présente invention au niveau des récepteurs $H_2$-gastriques de l'histamine a été évaluée dans le test de l'inhibition de la sécrétion provoquée par l'histamine sur la muqueuse isolée d'estomac de cobaye (P. Holton et J. Spencer, «J. Phisiol.», 1976, *255*; pp. 465-479).

L'activité antagoniste des composés de la présente invention au niveau des récepteurs $H_2$-cardiaques de l'histamine a été évaluée dans le test de l'inhibition de l'augmentation de la fréquence induite par l'histamine sur l'oreillette droite de cobaye (D. Reinhardt *et al.*, «Agents and Actions», 1974, *4*; pp. 217-221).

Le tableau I montre pour le composé de l'invention, sous forme de dichlorhydrate – indiqué dans les exemples ci-dessous par son numéro de code CM 57755 – et pour deux produits de référence, la 2-cyano-1-méthyl-3-[2[(5-méthylimidazol-4-yl)méthylthio]éthyl]guanidine, ci-après désignée par sa dénomination commune internationale cimétidine, et la N-[2[[5-[(diméthylamino)méthyl]furfuryl]thio]éthyl]-N'-méthyl-2-nitro-1,1-éthènediamine, ci-après désignée par sa dénomination commune internationale ranitidine, décrite dans la demande de brevet FR N° 2360587:

– la concentration de produit sous examen qui inhibe de 50% l'augmentation de la fréquence induite par l'histamine sur l'oreillette droite isolée de cobaye ($CI_{50}o$),

– la concentration de produit sous examen qui inhibe de 50% la sécrétion provoquée par l'histamine sur la muqueuse isolée d'estomac de cobaye ($CI_{50}m$),

– le rapport $CI_{50}m/CI_{50}o$.

*Tableau* →

→ *Tableau I*

| Composé | $CI_{50}o$ ($M \cdot 10^{-6}$) | $CI_{50}m$ ($M \cdot 10^{-6}$) | $CI_{50}m/CI_{50}o$ |
|---|---|---|---|
| Cimétidine | 0,4 | 6,6 | 16,5 |
| Ranitidine | 0,086 | 0,65 | 7,6 |
| CM 57755 | 4,3 | 9,2 | 2,13 |

La valeur très basse du rapport $CI_{50}m/CI_{50}o$ relative au composé de la présente invention, bien inférieure à celle des composés de référence, montre que le CM 57755 est capable d'antagoniser de manière plus sélective l'hypersécrétion gastrique que l'action chronotrope positive provoquées par l'histamine; à savoir, il montre une affinité plus élevée pour les récepteurs $H_2$-gastriques que pour les récepteurs $H_2$ -cardiaques.

L'activité antagoniste des produits de la présente invention vers les récepteurs $H_2$-gastriques de l'histamine a été confirmée dans le test de l'activité antisécrétoire basé sur l'antagonisme pour l'hypersécrétion provoquée par l'histamine chez le rat selon la méthode de Ghosh et Schild («Brit. J. Pharmacol.», 1958; *13*, 54). Selon ce test, on provoque une hypersécrétion acide gastrique par infusion intraveineuse d'une dose submaximale équivalent à 15 µmol/kg/h et l'on mesure la sécrétion gastrique par perfusion d'une solution physiologique à une vitesse constante dans l'estomac de l'animal. Dans ces conditions, le CM 57755 inhibe de 50% l'hypersécrétion à la dose de 0,9 mg/kg par voie intraveineuse; la $DL_{50}$ du produit par voie intraveineuse chez le rat est de 300 mg/kg.

L'activité antisécrétoire des produits de la présente invention a été évaluée chez le chat porteur de fistule gastrique selon la méthode de Emas *et al.* («Gastroenterology», 1960; *39*, 771) en utilisant, comme hypersécréteur, le dimaprit à la dose de 640 µg/kg/h. Dans ces conditions, le CM 57755, administré tant en perfusion veineuse que par voie intragastrique, antagonise de manière dose-dépendante l'hypersécrétion provoquée par le dimaprit. Son activité est comparable, sur base molaire, à celle de la cimétidine utilisée comme composé de référence. Le produit de la présente invention montre cependant une longue durée d'action après la suspension du traitement, tandis que l'effet du produit de référence est plus fugace.

Par rapport à leur degré d'activité, les composés de la présente invention sont peu toxiques et présentent un bon index thérapeutique.

La présente invention, selon un autre de ses aspects, se réfère ainsi à des compositions pharmaceutiques renfermant, en tant qu'ingrédients actifs, la thioalkylamide de l'acide nicotinique 1-oxyde de formule II ci-dessus, ainsi que ses sels d'addition acceptables du point de vue pharmaceutique.

Dans les compositions pharmaceutiques à action $H_2$-bloquante de la présente invention pour l'administration orale, sublinguale, sous-cutanée, intramusculaire, intraveineuse, transdermique ou rectale, les ingrédients actifs peuvent être administrées sous formes unitaires d'administration, en combinaison avec des supports pharmaceutiques classiques, aux animaux et aux êtres humains dans

le traitement de l'hypersécrétion gastrique et de l'ulcère peptique.

Parmi les formes unitaires d'administration appropriées, il y a les formes d'administration par voie orale telles que les comprimés, les gélules, les poudres, les granules et les solutions ou suspensions orales et les formes d'administration sublinguale, de même que les formes d'administration parentérale utiles pour une administration sous-cutanée, intramusculaire ou intraveineuse.

Afin d'obtenir l'effet $H_2$-bloquant désiré, la dose de principe actif peut varier entre 1 et 100 mg/kg de poids et par jour, de préférence de 10 à 50 mg/kg de poids et par jour.

Chaque dose unitaire peut contenir de 10 à 1000 mg d'ingrédient actif en combinaison avec un support pharmaceutique. Cette dose unitaire peut être administrée 1 à 4 fois par jour.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de sucrose ou d'autres matières appropriées ou encore on peut les traiter de telle sorte qu'ils aient une activité prolongée ou retardée et qu'ils libèrent continuellement une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant éventuellement acalorique, du méthylparaben et du propylparaben comme antiseptiques, ainsi qu'un agent donnant du goût et un colorant approprié.

Les poudres ou les granulats dispersibles dans l'eau peuvent contenir l'ingrédient actif en mélange avec des agents de dispersion ou des agents mouillants, ou des agents de mise en suspension, tels que la polyvinylpyrrolidone et similaires, de même qu'avec des édulcorants ou des correcteurs du goût.

Pour une application rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Les exemples suivants illustrent l'invention sans toutefois la limiter.

*Exemple 1:*

On ajoute par portions, sous agitation et à la température de 0 à 5°C, 9 g de chlorhydrate du chlorure de nicotinoyle 1-oxyde à une solution de 8,6 g de 2-(5-diméthylaminométhylfuran-2-ylméthylthio)éthylamine et 12,2 g de 4-diméthylaminopyridine dans 80 ml de chlorure de méthylène. On agite le mélange 30 min à 0,5°C et 30 min à la température ambiante, puis on filtre et on évapore le solvant sous pression réduite. On reprend le résidu avec 80 ml d'isopropanol et on l'acidifie avec de l'acide chlorhydrique en isopropanol. On refroidit avec de l'eau glacée et, 30 min après, on filtre. On obtient ainsi 15,5 g de dichlorhydrate de N-[2-(5-diméthylaminométhylfuran-2-ylméthylthio)éthyl]-3-pyridinecarboxamide 1-oxyde (CM 57755) qui, après cristallisation dans de l'éthanol à 95%, fond à 160-162°C.

*Exemple 2:*

A une solution de 6,4 g de 2-(5-diméthylaminométhylfuran-2-ylméthylthio)éthylamine, 12,7 ml de diisopropylamine dans 50 ml de chlorure de méthylène, on ajoute par portions, à 0,5°C et sous agitation, 8,7 g de chlorhydrate du chlorure de nicotinoyle 1-oxyde. On abandonne le mélange réactionnel 3 h à 0-5°C, puis on évapore à sec sous pression réduite, on reprend le résidu avec 40 ml d'eau et on l'acidifie avec de l'acide chlorhydrique. On lave la solution acide deux fois avec 30 ml d'acétate d'éthyle, et on la filtre avec du charbon et on la rend nettement basique avec de l'hydroxyde de sodium concentré. On extrait le produit avec de l'acétate d'éthyle, on sèche la solution organique sur du sulfate de sodium anhydre et on l'évapore à sec. On reprend le résidu avec une petite quantité d'éther diéthylique. Après filtration des cristaux et recristallisation de l'acétate d'éthyle, on obtient 5,2 g de N-[2-(5-diméthylaminométhylfuran-2-ylméthylthio)éthyl]-3-pyridinecarboxamide 1-oxyde, CM 57862; p.f. 76 à 78°C.

*Exemple 3:*

A une solution de 1 g de N-[2-(5-diméthylaminométhylfuran-2-ylméthylthio)éthyl]-3-pyridinecarboxamide 1-oxyde dans 15 ml d'éthanol, on ajoute une solution de 0,3 g d'acide oxalique dans 10 ml d'éthanol. Le sel qui précipite est filtré, séché et cristallisé de 10 ml d'éthanol à 95%. On obtient ainsi 1 g d'oxalate de N-[2-(5-diméthylaminométhylfuran-2-ylméthylthio)éthyl]-3-pyridinecarboxamide 1-oxyde, CM 57874; p.f. 105 à 107°C.

*Exemple 4:*

A une solution de 10,2 g de dichlorhydrate de N-[2-(5-diméthylaminométhylfuran-2-ylméthylthio)éthyl]-3-pyridinecarboxamide 1-oxyde dans 15 ml d'eau, on ajoute de l'hydroxyde de sodium jusqu'à réaction nettement basique. On extrait avec de l'acétate d'éthyle contenant 10% d'éthanol, on sèche la phase organique sur du sulfate de sodium anhydre et on l'évapore à sec. On obtient ainsi un liquide visqueux qui cristallise lentement. On triture le produit dans de l'éther diéthylique, on filtre et on sèche. Après cristallisation de 30 ml d'acétate d'éthyle, on obtient 6,8 g de N-[2-(5-diméthylaminométhylfuran-2-ylméthylthio)-éthyl]-3-pyridinecarboxamide 1-oxyde, identique au produit de l'exemple 2.

*Exemple 5:*

Comprimés ayant la composition suivante:

| | |
|---|---|
| CM 57755 | 100 mg |
| lactose | 70 mg |
| fécule de pommes de terre | 40 mg |
| polyvinylpyrrolidone | 8 mg |
| stéarate de magnésium | 2 mg |

On humecte le mélange de la substance active avec le lactose et la fécule de pommes de terre d'une solution alcoolique de polyvinylpyrrolidone à 15%, on fait passer le granulé formé à travers un tamis de 1 mm, on le mélange avec le stéarate de magnésium et on forme des comprimés par compression. Poids d'un comprimé: 220 mg.

*Exemple 6:*

On recouvre de façon connue les comprimés fabriqués comme décrit à l'exemple 5 d'un enrobage pour dragées consistant essentiellement en sucre et talc, et on polit les dragées finies par de la cire d'abeilles. Poids d'une dragée: 300 mg.

*Exemple 7:*

Gélules ayant la composition suivante:

| | |
|---|---|
| CM 57755 | 200 mg |
| amidon de maïs | 90 mg |
| talc | 10 mg |

On mélange intimement le principe actif et les excipients et l'on introduit le mélange ainsi obtenu dans des gélules de gélatine de dimension 1. Contenu d'une gélule: 300 mg.

*Exemple 8:*

Suppositoires ayant la composition suivante:

| | |
|---|---|
| CM 57755 | 300 mg |
| masse pour suppositoire (Witespol W45) | 1450 mg |

On met en suspension la substance active finement pulvérisée dans la masse pour suppositoires à 37°C et on verse le mélange dans des moules légèrement refroidis au préalable. Poids d'un suppositoire: 1750 mg.

*Exemple 9:*

Comprimés ayant la composition suivante:

| | |
|---|---|
| CM 57755 | 300 mg |
| cellulose microcristalline | 100 mg |
| amidon de maïs | 50 mg |
| polyvinylpyrrolidone | 10 mg |
| stéarate de magnésium | 5 mg |

On mélange le principe actif avec les trois excipients, on fait passer le mélange obtenu à travers un tamis de 0,5 mm, puis on ajoute le stéarate de magnésium lubrifiant et on comprime.

De la même façon, on prépare des comprimés contenant 500 mg de CM 57755.

*Exemple 10:*

Gélules ayant la composition suivante:

| | |
|---|---|
| CM 57755 | 350 mg |
| amidon de maïs | 140 mg |
| talc | 10 mg |

On mélange intimement le principe actif et les excipients et l'on introduit le mélange ainsi obtenu dans des gélules de gélatine de dimension 0.

De la même façon, on prépare des gélules contenant 500 mg de CM 57755.

**Revendications** pour les Etats contractants BE, CH, DE, FR, GB, IT, LI, LU, NL, SE

1. Thioalkylamide de formule:

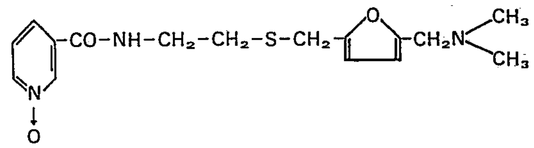

ainsi que ses sels pharmaceutiquement acceptables.

2. Dichlorhydrate de N-[2-(5-diméthylaminométhylfuran-2-ylméthylthio)éthyl]-3-pyridinecarboxamide 1-oxyde.

3. Procédé pour la préparation des composés selon la revendication 1, caractérisé en ce qu'on traite la 2-(5-diméthylaminométhylfuran-2-ylméthylthio)éthylamine de formule:

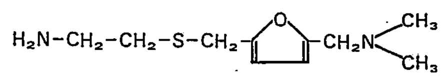

avec un dérivé fonctionnel de l'acide nicotinique 1-oxyde dans un solvant organique à une température entre 0°C et la température d'ébullition du solvant employé, et on transforme éventuellement le produit ainsi obtenu dans ses sels pharmaceutiquement acceptables.

4. Composition pharmaceutique renfermant, en tant que principe actif, un composé selon les revendications 1 et 2.

5. Composition pharmaceutique à action $H_2$-bloquante renfermant, par chaque unité de dosage, de 10 à 1000 mg d'un produit selon les revendications 1 et 2 en mélange avec un excipient pharmaceutique.

**Revendications** pour l'Etat contractant: AT

1. Procédé pour la préparation du N-[2-(5-diméthylaminométhylfuran-2-ylméthylthio)éthyl]-3-pyridinecarboxamide 1-oxyde de formule:

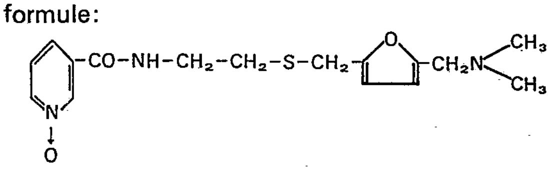

et de ses sels pharmaceutiquement acceptables, caractérisé en ce qu'on traite la 2-(5-diméthylaminométhylfuran-2-ylméthylthio)éthylamine de formule:

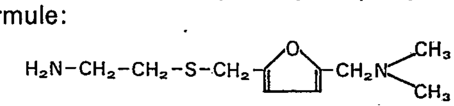

avec un dérivé fonctionnel de l'acide nicotinique 1-oxyde dans un solvant organique à une température entre 0°C et la température d'ébullition du solvant employé et en ce qu'on transforme éventuellement le produit ainsi obtenu dans ses sels pharmaceutiquement acceptables.

2. Procédé selon la revendication 1, caractérisé en ce que, comme dérivé fonctionnel de l'acide nicotinique 1-oxyde, on utilise le chlorhydrate du chlorure de nicotinoyle 1-oxyde.

3. Procédé selon les revendications 1 et 2, caractérisé en ce que la réaction est conduite en présence d'un accepteur de protons.

4. Procédé selon les revendications 1 à 3, caractérisé en ce que le N-[2-(5-diméthylaminométhylfuran-2-ylméthylthio)éthyl]-3-pyridinecarboxamide 1-oxyde est isolé sous forme de dichlorhydrate.

5. Procédé selon les revendications 1 à 4, caractérisé en ce qu'on libère le N-[2-(5-diméthylaminométhylfuran-2-ylméthylthio)-éthyl]-3-pyridinecarboxamide 1-oxyde de ses sels à l'aide d'un hydroxyde ou d'un carbonate alcalin.

**Patentansprüche** für die Vertragsstaaten BE, CH, DE, FR, GB, IT, LI, LU, NL, SE

1. Thioalkylamid der Formel:

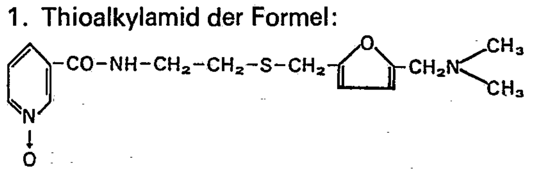

sowie seine pharmazeutisch akzeptablen Salze.

2. N-[2-(5-Dimethylaminomethylfuran-2-yl-methylthio)äthyl]-3-pyridincarboxamid-1-oxid-dihydrochlorid.

3. Verfahren zur Herstellung der Verbindungen nach Anspruch 1, dadurch gekennzeichnet, dass man 2-(5-Dimethylaminomethylfuran-2-ylmethylthio)-äthylamin der Formel:

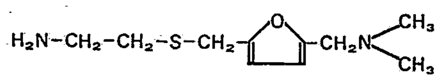

mit einem funktionellen Derivat des Nikotinsäure-1-oxids in einem organischen Lösungsmittel bei einer Temperatur zwischen 0°C und der Siedetemperatur des verwendeten Lösungsmittels behandelt und gegebenenfalls das so erhaltene Produkt in seine pharmazeutisch akzeptablen Salze überführt.

4. Pharmazeutische Zusammensetzung, die als Wirkstoff eine Verbindung nach den Ansprüchen 1 und 2 enthält.

5. Pharmazeutische Zusammensetzung mit $H_2$-blockierender Wirkung, die pro Dosiseinheit 10 bis 1000 mg eines Produktes nach den Ansprüchen 1 und 2 in Mischung mit einem pharmazeutischen Exzipienten enthält.

**Patentansprüche** für den Vertragsstaat: AT

1. Verfahren zur Herstellung von N-[2-(5-Dimethylaminomethylfuran-2-yl-methylthio)-äthyl]-3-pyridincarboxamid-2-oxid der Formel:

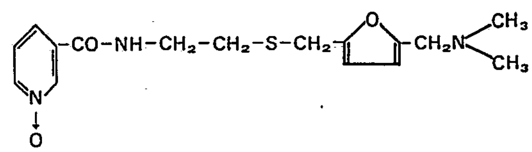

und seiner pharmazeutisch akzeptablen Salze, dadurch gekennzeichnet, dass man 2-(5-Dimethylaminomethylfuran-2-yl-methylthio)-äthylamin der Formel:

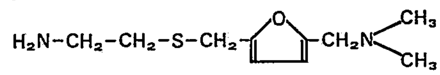

mit einem funktionellen Derivat des Nikotinsäure-1-oxids in einem organischen Lösungsmittel bei einer Temperatur zwischen 0°C und der Siedetemperatur des verwendeten Lösungsmittels behandelt und gegebenenfalls das so erhaltene Produkt in seine pharmazeutisch akzeptablen Salze überführt.

2. Verfahren nach Anspruch 1, dadurch gekennzeichnet, dass man als funktionelles Derivat des Nikotinsäure-1-oxids das Hydrochlorid von Nikotinoylchlorid-1-oxid verwendet.

3. Verfahren nach den Ansprüchen 1 und 2, dadurch gekennzeichnet, dass die Reaktion in Gegenwart eines Protonenakzeptors durchgeführt wird.

4. Verfahren nach den Ansprüchen 1 bis 3, dadurch gekennzeichnet, dass N-[2-(5-Dimethylaminomethylfuran-2-yl-methylthio)-äthyl]-3-pyridincarboxamid-1-oxid in der Form des Dihydrochlorids isoliert wird.

5. Verfahren nach den Ansprüchen 1 bis 4, dadurch gekennzeichnet, dass man N-[2-(5-Dimethylaminomethylfuran-2-yl-methylthio)-äthyl]-3-pyridincarboxamid-2-oxid mit Hilfe eines Alkalihydroxids oder eines -carbonats aus seinen Salzen freisetzt.

**Claims** for the Contracting States BE, CH, DE, FR, GB, IT, LI, LU, NL, SE

1. Thioalkylamide of formula:

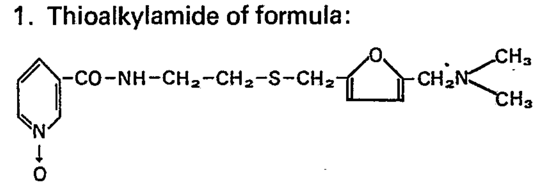

and its pharmaceutically acceptable salts.

2. N-[2-(5-dimethylaminomethylfuran-2-ylmethylthio)ethyl]-3-pyridine-carboxamide 1-oxyde dihydrochloride.

3. Process for the preparation of the compounds according to claim 1, characterized in that 2-(5-dimethylaminomethylfuran-2-ylmethylthio)-ethylamine of formula:

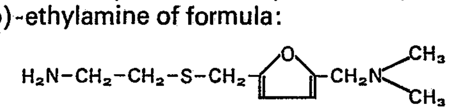

is treated with a functional derivative of the 1-oxide nicotinic acid in an organic solvent at a temperature between 0°C and the temperature of boiling of the solvent employed, and the product

thus obtained is possibly converted into its pharmaceutically acceptable salts.

4. Pharmaceutical composition, comprising, as active ingredient, a compound according to claims 1 and 2.

5. Pharmaceutical composition with a $H_2$-blocking effect comprising, per unitary dose, from 10 to 1000 mg of an ingredient according to claims 1 and 2 in combination with a pharmaceutical support.

**Claims** for the Contracting State: AT

1. Process for preparing N-[2-(5-dimethylaminomethylfuran-2-ylmethylthio)ethyl]-3-pyridinecarboxamide 1-oxide of formula:

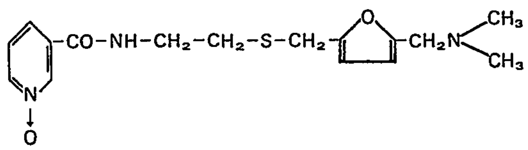

and its pharmaceutically acceptable salts, characterized in that 2-(5-dimethylaminomethylfuran-2-ylmethylthio)-ethylamine of formula:

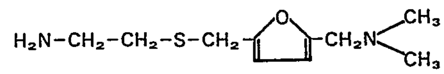

is treated with a functional derivative of the 1-oxide nicotinic acid in an organic solvent at a temperature between 0°C and the temperature of boiling of the solvent employed, and the product thus obtained is possibly converted into its pharmaceutically acceptable salts.

2. The process according to claim 1, characterized in that the hydrochloride of 1-oxide nicotinoyl chloride is used as functional derivative of 1-oxide nicotinic acid.

3. The process according to claims 1 and 2, characterized in that the reaction is conducted in the presence of a proton acceptor.

4. The process according to claims 1 to 3, characterized in that the N-[2-(5-dimethylaminomethylfuran-2-ylmethylthio)ethyl]-3-pyridinecarboxamide 1-oxide is isolated in the form of dihydrochloride.

5. The process according to claims 1 to 4, characterized in that the N-[2-(5-dimethylaminomethylfuran-2-ylmethylthio)ethyl]-3-pyridinecarboxamide 1-oxide is released from its salts with the aid of a hydroxide or an alkaline carbonate.